# EUROPEAN PATENT APPLICATION

(11) **EP 4 105 316 A1**
(43) Date of publication of application: **21.12.2022**
(21) Application number: 20918232.8
(22) Date of filing: 10.02.2020
(51) Int. Cl.: C12M 3/00, C12N 5/071

(54) **CELL CULTURE MANAGEMENT SYSTEM**

(71) Applicant: Integriculture Inc., Tokyo 113-0033 (JP)
(72) Inventor: KAWASHIMA, Ikko, Tokyo 113-0033 (JP); HANYU, Yuki, Tokyo 113-0033 (JP)
(74) Representative: Grünecker Patent- und Rechtsanwälte PartG mbB
(86) International application number: PCT/JP2020/005189
(87) International publication number: WO 2021/161397

(57) **Abstract**

The present invention provides cell culture management systems. A cell culture management system according to the present invention includes an output data server provided in a first culture device for culturing a first kind of cells; a customer server provided in a second culture device for culturing a second kind of cells; and a cell management server provided in a cell management compartment where the second kind of cells are stored, in which the output data server, the customer server, and the cell management server are each connected to and in bidirectional communication with a data management device.

## Description

### Technical field

The present invention relates to cell culture management systems.

### Background art

Various kinds of culture devices and culture methods have been developed to grow internal organs or maintain their functions for a long time. For example, WO2017/191691 discloses growth induction systems, growth induction control devices, growth induction control methods, and growth induction control programs with which growth can be induced without the need to add cytokines for each of different stages of growth.

### Problems to be solved by the invention

Nevertheless, no management system for the cell culture systems as a whole when they are individually installed has ever existed.

Therefore, an object of the present invention is to provide novel cell culture management systems.

### Means to solve the problems

An aspect of the present invention is a cell culture management system including: a data management device; an output data server provided in a first culture device for culturing a first kind of cells; a customer server provided in a second culture device for culturing a second kind of cells; and a cell management server provided in a cell management compartment where the second kind of cells are stored, in which: the data management device is connected in bidirectional communication with each of the output data server, the customer server, and the cell management server; the output data server has: a transmitter for transmitting a first protocol for culturing the first kind of cells to the data management device; the customer server has: one or more transmitters for transmitting order information for obtaining the second kind of cells and location information of the second culture device to the data management device; and a receiver for receiving a second protocol for culturing the second kind of cells from the data management device; the data management device has: a receiver for receiving the first protocol from the output data server; and a receiver for receiving the order information and the location information of the second culture device from the customer server; and one or more memorizing units for memorizing the first protocol received from the output data server, the second protocol, and the order information and the location information of the second culture device received from the customer server; and the cell management server has: a receiver for receiving the order information and the location information of the second culture device from the data management device. The output data server may have a receiver for receiving a third protocol for culturing a third kind of cells from the data management device. The customer server may have a transmitter for transmitting an measured culture data obtained from a cell sensor disposed in the second culture device to the data management device; and the data management device may have a receiver for receiving the measured culture data transmitted from the customer server; and a memorizing unit for memorizing the measured culture data received from the customer server. The cell management server may have a memorizing unit for memorizing inventory information for cells present in the cell management compartment; and a transmitter for transmitting the inventory information to the data management device; and the data management device may have a receiver for receiving the inventory information transmitted from the cell management server; and a memorizing unit for memorizing the received inventory information.

Another aspect of the present invention is a cell culture management system including: an output data server provided in a first culture device for culturing a first kind of cells; a customer server provided in a second culture device for culturing a second kind of cells; and a cell management server provided in a cell management compartment where the second kind of cells are stored, in which: the output data server, the customer server, and the cell management server are each connected in bidirectional communication with a data management device; the output data server has: a transmitter for transmitting a first protocol for culturing the first kind of cells to the data management device; the customer server has: one or more transmitters for transmitting order information for obtaining the second kind of cells and location information of the second culture device to the data management device; and a receiver for receiving a second protocol for culturing the second kind of cells from the data management device; and the cell management server has: a receiver for receiving the order information and the location information of the second culture device from the data management device. The output data server may have a receiver for receiving a third protocol for culturing a third kind of cells from the data management device. The customer server may have a transmitter for transmitting an measured culture data obtained from a cell sensor disposed in the second culture device to the data management device; and the data management device may have a receiver for receiving the measured culture data transmitted from the customer server; and a memorizing unit for memorizing the measured culture data received from the customer server. The cell management server may have a memorizing unit for memorizing inventory information for cells present in the cell management compartment; and a transmitter for transmitting the inventory information to the data management device; and the data management device may have a receiver for receiving the inventory information transmitted from the cell management server; and a memorizing unit for memorizing the received inventory information.

### Brief description of the drawings

Fig. 1 is a flow chart showing an operation of a cell culture system in a basic process, which is an embodiment according to the present invention.
Fig. 2 is a schematic diagram of a cell culture system in the basic process, which is an embodiment according to the present invention. The broken lines indicate flows of information, and the solid lines indicate flows of objects;
Fig. 3 is a schematic diagram of a cell culture system in novel protocol development, which is another embodiment according to the present invention. The broken lines indicate flows of information, and the solid lines indicate flows of objects.
Fig. 4 is a schematic diagram of a cell culture system in feedbacks of culture conditions, which is another embodiment according to the present invention. The broken lines indicate flows of information, and the solid lines indicate flows of objects.
Fig. 5 is a schematic diagram of a cell culture system in inventory management, which is another embodiment according to the present invention. The broken line indicates the flow of information, and the solid line indicates a flow of an object.

### Modes for carrying out the invention

Preferred embodiments of the present invention are described in detail with reference to the accompanying drawing, but the present invention is not necessarily limited thereto. Objects, characteristics, advantages, and ideas of the present invention are apparent to a person skilled in the art from the description of the present specification, and the person skilled in the art can easily reproduce the present invention from the description of the present specification. Modes for carrying out the invention, specific examples thereof and so forth, which are described below, provide preferable embodiments of the present invention. They are described for the purpose of illustration or explanation, and thus the present invention is not limited thereto. It is apparent to a person skilled in the art that various alterations and modifications can be made on the basis of the description of the present specification within the spirit and the scope of the present invention disclosed in the present specification.

### == Cell culture management system ==

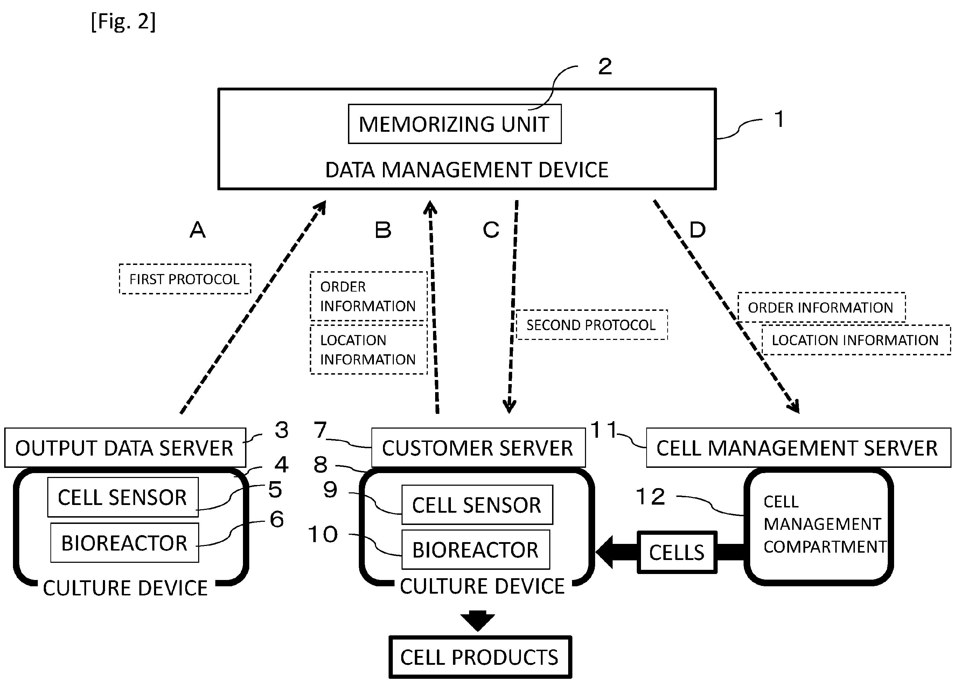

A cell culture management system according to an embodiment of the present invention includes an output data server provided in a first culture device for culturing a first kind of cells; a customer server provided in a second culture device for culturing a second kind of cells; and a cell management server provided in a cell management compartment where the second kind of cells are stored, in which the output data server, the customer server, and the cell management server are each connected to and in bidirectional communication with a data management device external to the system; the output data server has a transmitter for transmitting a first protocol for culturing the first kind of cells to the data management device; the customer server has one or more transmitters for transmitting order information for obtaining the second kind of cells and location information of the second culture device to the data management device, and a receiver for receiving a second protocol for culturing the second kind of cells from the data management device; and the cell management server has a receiver for receiving the order information and the location information of the second culture device from the data management device. Here, the data management device has a receiver for receiving the first protocol from the output data server; a receiver for receiving the order information and the location information of the second culture device from the customer server; and one or more memorizing units that memorize the first protocol received from the output data server, the second protocol, and the order information, and the location information of the second culture device received from the customer server. A specific embodiment of this system is described below with reference to Fig. 2.

### <Data management device>

A data management device 1 is connected in bidirectional communication with one or more output data servers 3, one or more customer servers 7, and one or more cell management servers 11 via a network such as the Internet, a local area network (LAN), a wide area network (WAN), or a leased line. The data management device 1 has, at least, a receiver for receiving a protocol or protocols from the output data server 3 and a receiver for receiving order information and location information of the culture device 8 from the customer server 7, and also has a memorizing unit that stores the received data. The received data are preferably stored in a database.

The data management device 1 may be external to the cell culture management system like a cloud device, or it may be managed as a component of the cell culture management system.

The data management device 1 may be a simple device consisting of a transceiver and the memorizing unit 2 only for holding data. Alternatively, it may have a data processor other than the transceiver and the memorizing unit 2 to perform various processing on the data.

### <Output data server>

The output data server 3 is included in a culture device 4 for developing new protocols for culturing cells. The output data server 3 has a transmitter for transmitting a new protocol to the data management device 1. The protocol may include, for example, information for cell culture, such as the type of cell, culture conditions (e.g., the temperature and CO₂ concentration), the type of medium to be used, a reference value for a cell marker to be monitored over time, a reference value for the pH of a medium to be evaluated over time, and/or a reference value for a cell density to be monitored over time.

The culture device 4 is not limited and any known culture device may be used. For example, the culture device 4 may consist of only an incubator for culturing cells; however, it may also include a bioreactor 6. The bioreactor 6 includes, for example, one or more culture chambers for culturing cells and producing cell products, a control unit for regulating cell culture, and a memory for memorizing culture protocols including culture conditions. When two or more culture chambers are used, each culture chamber may have an individual culture medium cycling mechanism to cycle the flow of the culture medium in the culture chamber, or a culture medium circulator may be included when the culture chambers are connected to each other to allow the culture medium to circulate between them. The control unit for regulating culture may regulate the flow rate of a culture medium and the direction of cycling a culture medium using the culture medium cycling mechanism.

The culture device 4 preferably has a cell sensor 5 that can automatically detect the condition of the cell culture over time. The cell sensor 5 can preferably obtain physical, chemical, and biological characteristics and/or properties of cells, culture media or cell products in a culture chamber over time, including their weight, temperature, viscosity, color (absorbance), pH, sugar content, salinity, density, and type. The cell sensor 5 may be controlled by the control unit. The conditions detected using the cell sensor 5 may be stored in a memory.

Furthermore, the data management device 1 may have a transmitter for transmitting culture protocols to the output data server 3, while the output data server 3 may have a receiver for receiving culture protocols from the data management device 1. Culture protocol creators may create an entirely new protocol, but having a way to receive protocols enables the creators to create a new protocol by modifying an existing culture protocol.

### <Customer server>

The customer server 7 is included in a culture device 8 for a customer to culture cells. The culture device 8 may have a similar structure to the culture device 4 related to the output data server 3. For example, the culture device 8 may include a bioreactor 10 and a cell sensor 9 which are similar to those in the culture device 4. Particularly, when a novel protocol is developed for the culture device 4 related to the output data server 3, it is preferable that the culture devices 4 and 8 are exactly of the same model in order to use identical conditions.

For customers, the culture's purposes are not limited. For example, cells for food, medical or material use may be grown, or a substance may be produced using cells.

The customer server 7 has a transmitter for transmitting order information and location information of the culture device 8 to the data management device 1. The content of the order information is not limited as long as the customer's purpose is described. For example, when a kind of cells is needed, the order information may be the desired cell's name, or when a cell product is needed, it may be the cell product's name. Furthermore, besides the name of the desired cells or cell products, information for usually ordering a commercial item may be included. For example, by including information on when the cells or cell products are required, it is feasible to estimate when the cells will be delivered from a cell management compartment. The location information of the culture device 8 denotes where the culture device 8 in question is. The location can be recognized using, for example, its address.

The customer server 7 also has a receiver for receiving a protocol or protocols for producing the desired kind of cells or cell products from the data management device 1. The protocols received from the data management device 1 may be stored in a memory included in a bioreactor.

In addition, the customer server 7 may have a transmitter for transmitting, to the data management device 1, measured culture data including a measured value corresponding to a reference value for a protocol (e.g., reference values for pH and cell density) obtained over time from a cell sensor in the bioreactor while the cells are being cultured in a culture device 8 or from a cell sensor at the end of the culture. By having such means, when similar cell products are produced in two or more bioreactors, variations between the products can be detected and managed. In this situation, the data management device may have a receiver for receiving the measured culture data and a memorizing unit for memorizing the measured culture data. The memorizing unit may be the same as or different from a memorizing unit for storing other data.

When two or more culture chambers are used in the bioreactor 10, the protocol may also include information on where culture cells are placed at the beginning (e.g., a culture chamber number in the bioreactor) and instructions for changing conditions of circulating culture media between or among culture chambers over time.

### <Cell management server>

The cell management server 11 is included in a cell management compartment 12 where cells are stored, and has a receiver for receiving, from the data management device 1, order information transmitted from customers using their local customer servers, and location information of their local culture devices 8 for customers to culture the desired cells. Additionally, the cell management server 11 may receive a culture protocol or protocols as well as delivery item information, which are associated with the order information from the data management device 1. The delivery item information denotes an item or items to be delivered to a customer in response to an order from that customer and includes information regarding the materials needed for culture (e.g., consumables such as culture medium). Based on the information, it becomes feasible to deliver the item(s) requested by the customer to the place where the culture device 8 is situated.

The cell management server 11 may have a transmitter for transmitting inventory information for different cells stored in a cell management compartment or the local cell management server 11 to the data management device 1. A cell administrator accessing the data management device 1 may refill a low or no inventory of cells by purchasing cells or growing the cells by himself.

The cell management compartment may include a warehouse, a refrigerator, a freezer, and/or a culture chamber for storing cells and distributing them when instructed to do so.

### == Cell culture management method ==

How cells can be managed using the aforementioned cell culture management system is described in detail using several embodiments as examples.

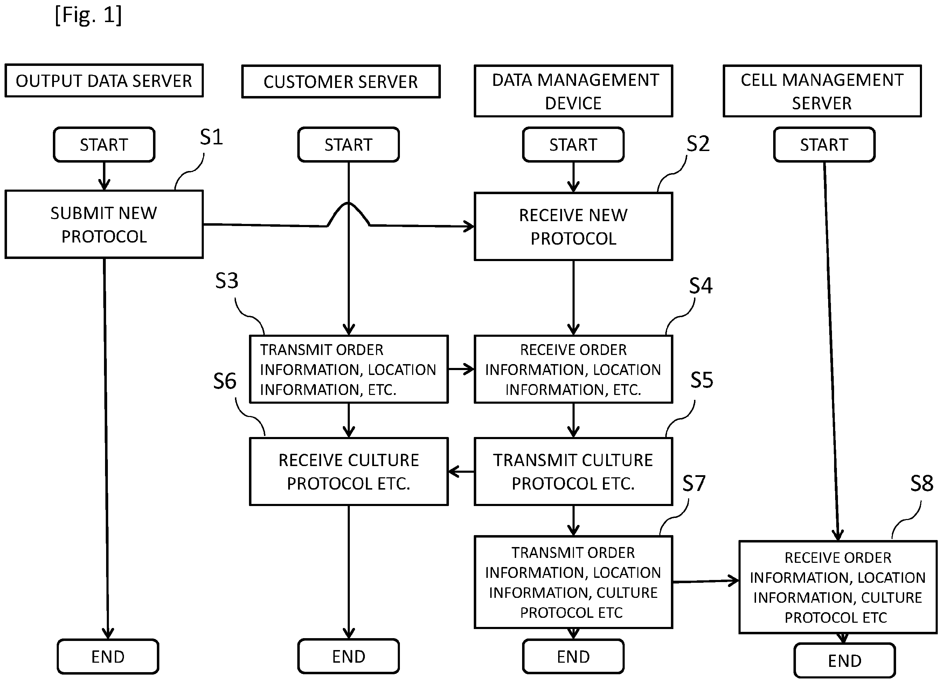

### (1) Basic process (Figs. 1 and 2)

A basic process in the cell culture management method using this management system is described using Figs. 1 and 2.

When a culture protocol creator submits a culture protocol in which the names and IDs of cells and/or cell products are associated with a related cell culture method, to the data management device 1 via an output data server 3 (S1), the data management device 1 receives the data transmitted from the output data server 3 and stores it in a memorizing unit 2 (S2)(A). The cell culture method has been developed by the culture protocol creator, and on the development, the culture conditions in the local bioreactor 6 detected by the local cell sensor 5 may be included in the culture protocol.

A customer places an order to the data management device 1 to obtain the desired cells using the local customer server 7. At that time, when the location information of a culture device 8 where cells are produced and other information are transmitted to the data management device 1 with the order information (S3), the data management device 1 receives the data transmitted from the customer server 7 and stores the data in the memorizing unit 2 (S4)(B). The order information may be related to the cells which the customer aim at culturing, or related to the cell products which the customer aim at producing using the cultured cells. The information in this case is preferably the name of the cells or cell products, but it may be an ID when the data management device manages the cultured cells, cell products, or culture protocols by their IDs and the customer is aware of the IDs.

When the data management device 1 receives the order from the customer server 7 and delivers a culture protocol for culturing cells (S5), the customer receives, through the customer server 7, the data transmitted from the data management device 1 (S6)(C). For this, the data management device 1 may automatically deliver a culture protocol matching the order information in response to its receipt, or the customer may select and download a suitable culture protocol from the data in the data management device 1.

The data management device 1 that has received the order from the customer delivers the order information from the customer, the location information of the customer's culture device, and some other information (S7), and a cell management server 11 in a cell management compartment 12 receives the data from the data management device 1 (S8)(D). For receiving data by the cell management server 11, the cell management server 11 may, for example, retrieve the data from the data management device 1 at regular intervals, or an administrator of the cell management compartment 12 may access the data management device 1 to get the data at his/her convenient time. Alternatively, the data management device 1 that has received the order may automatically transmit the data to the cell management server 11, along with, for example, the location information of the customer's culture device. The administrator of the cell management compartment 12 subsequently delivers the items requested by the customer to the customer's culture device 8 according to the received data.

The customer, using the delivered items received from the cell management compartment 12, places the cells in the culture chamber of the bioreactor 10 according to the received culture protocol and initiates culture to grow the desired cells. Alternatively, cell products generated from the cell growth may be collected.

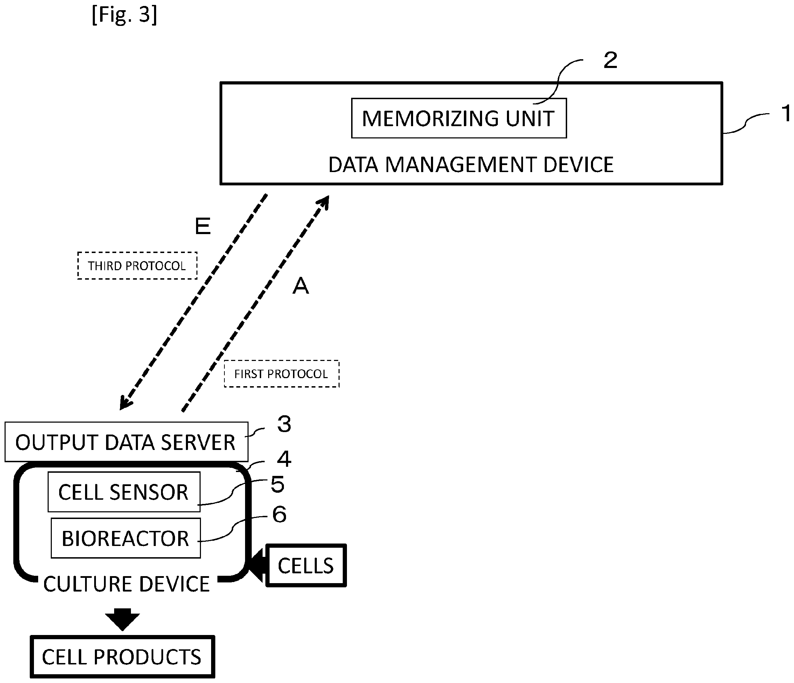

### (2) Creation of novel protocols (Fig. 3)

An embodiment in which an output data server 3 transmits a novel protocol to the data management device 1 is described with reference to Fig. 3.

A culture protocol creator can develop an entirely new protocol and submit it to the data management device 1 with a new management number (A). Alternatively, the creator may receive an existing protocol "a" from the data management device 1 through an output data server 3 (E), investigate the protocol using a culture device 4, develop a new protocol "b" from the protocol "a", and then submit the protocol "b" to the data management device 1 with a new management number (A).

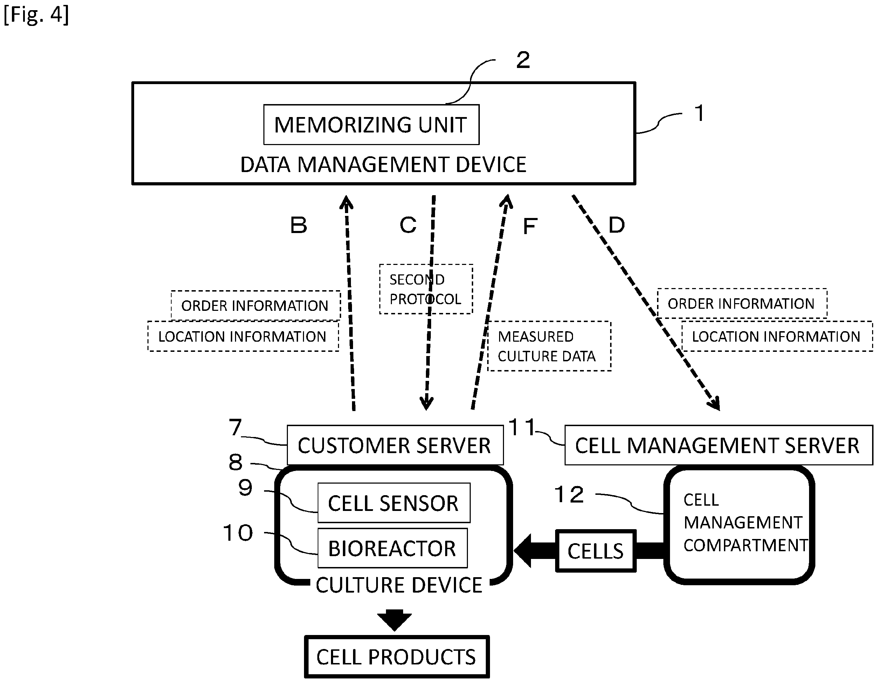

### (3) Feedback from customers (Fig. 4)

An embodiment in which a customer server 7 feeds back evaluated culture data to the data management device 1 is described with reference to Fig. 4.

A customer accesses the data management device 1 using the customer's local customer server 7 and places an order. At that time, the order information and the location information of the culture device 8 where the desired cells are cultured are transmitted to the data management device 1 (B). The data management device 1 receives the information and memorizes it in the memorizing unit 2. In response to this, the customer server 7 receives a culture protocol for culturing the desired cells from the data management device 1 (C).

When a cell management server 11 included in a cell management compartment 12 receives the order information from the customer, the location information of the customer's culture device, or some other information transmitted from the data management device 1, an administrator of the cell management compartment 12 delivers the cells to the customer's culture device 8 according to the received data (D).

The customer may load the received protocol into the bioreactor 10 and starts culturing the cells in a certain culture chamber in the bioreactor 10. Then, the customer server 7 may transmit, to the data management device 1, measured culture data including a measured value corresponding to a reference value for a protocol (e.g., reference values of pH and cell density) obtained over time from a cell sensor 9 in the culture device 8 while the desired cells are being cultured in the culture device 8 or from a cell sensor at the end of the culture of the desired cells (F). The data management device 1 receives the measured culture data and may store it in the memorizing unit 2.

By managing such measured culture data in the data management device 1, culture protocol creators can freely utilize the measured culture data in developing protocols.

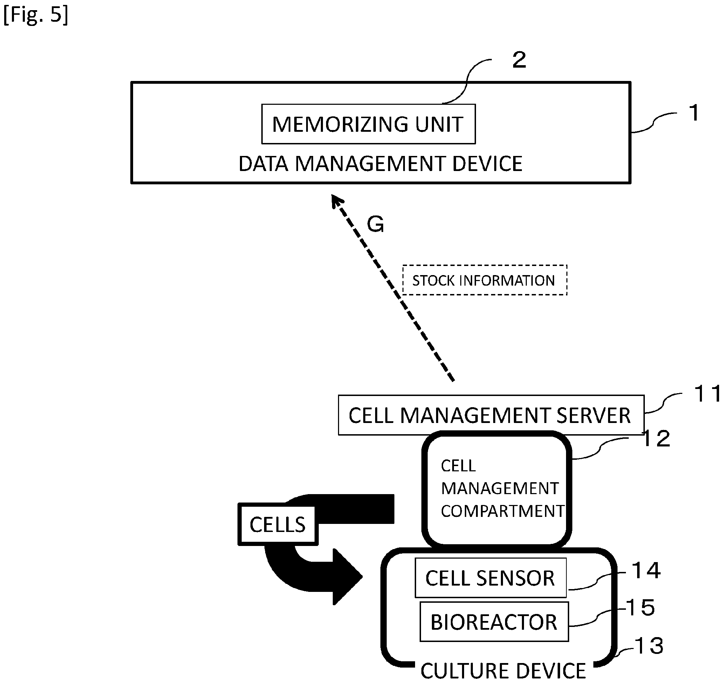

### (4) Inventory management (Fig. 5)

An embodiment in which the cell management server 11 manages an inventory of cells is described with reference to Fig. 5.

The cell management server 11 may transmit cell inventory information to the data management device 1 (G). This may be achieved by periodically updating the cell inventory information. Alternatively, the inventory information may be updated only when the content of the cell inventory information has been changed at the cell management server 11. The data management device 1 manages the inventory information as a database in the memorizing unit 2. When new inventory information is transmitted from the cell management server 11, the data management device 1 receives it and updates the database in the storage device 2. The inventory information may include not only the inventory quantity, but also expiration date information and/or some other information.

An administrator of the cell management compartment 12 accesses the data management device 1 and delivers new cells into the cell management compartment 12 for the kinds of cells that are low or no longer in stock, or past their use-by date. Alternatively, to ensure cell inventory, cells are grown using a culture device 13 included in the cell management compartment 12. The culture device 13 may be similar to or different from the culture device 4 of the output data server 3 or the culture device 8 of a customer server 7. It is, however, preferable that the culture device 13 is of the same type and has a cell sensor 14 and a bioreactor 15.

### == Specific example of operation using a cell culture management system ==

This section describes exemplified operations of the cell culture management system as far as customers are concerned. These embodiments enable customers to obtain a culture protocol and cells appropriate for producing desired cells or cell products using a specific culture device by indicating desired kind of cells or cell products, and to produce cells or cell products with a high reproducibility by culturing cells with the obtained culture protocol.

### (1) The case that customers order cells

Customers are given a culture device for culturing desired cells in advance.

When a customer needs to culture cells, he/she accesses the data management device 1 using a customer server 7. The data management device 1 presents a list of cells that can be ordered and an order form as database. The customer selects a kind of cells from the list, clicks on the name or ID of the cell or writes the name or ID of the cell; simultaneously, fills the address of a culture device 8 where cells will be produced in the order form. Finally, the customer transmits these pieces of information to the data management device 1.

The customer may download a culture protocol associated with the ordered kind of cells. The culture protocol describes how to culture the cells of interest (e.g., type of medium, culture conditions, etc.).

Upon learning of the order from the data management device 1, an administrator of a cell management compartment 12 delivers items associated with that kind of cells and necessary for their culture to the customer, and the customer receives the delivered items. The customer can culture the desired cells using the received items in the culture device according to the downloaded culture protocol.

### (2) The case that customers order cell products

Customers are given in advance a culture device for culturing cells to generate desired cell products.

When a customer needs to generate cell products, he/she accesses the data management device 1 using a customer server 7. The data management device 1 presents a list of cell products that can be produced and an order form as database. The customer selects a kind of cell products from the list, clicks on the name or ID of the cell product or inputs the name or ID of the cell product; simultaneously, fills the address of a culture device 8 where the cell product will be produced in the order form. Finally, the customer transmits the information to the data management device 1.

The customer may download a culture protocol associated with the ordered kind of cell products. The culture protocol describes how to culture cells to produce the cell products of interest (e.g., type of cell, type of medium, culture conditions, etc.).

Upon learning of the order from the data management device 1, an administrator of a cell management compartment 12 delivers items associated with that kind of cell products and necessary for cell culture for their generation to the customer, and the customer receives the delivered items. The customer can generate the desired cell products by culturing given cells using the received items in the culture device according to the downloaded culture protocol.

### Denotation of symbols

- 1: data management device
- 2: memorizing unit
- 3: output data server
- 4: culture device
- 5: cell sensor
- 6: bioreactor
- 7: customer server
- 8: culture device
- 9: cell sensor
- 10: bioreactor
- 11: cell management server
- 12: cell management compartment
- 13: culture device
- 14: cell sensor
- 15: bioreactor

### Industrial applicability

The present invention made it possible to provide novel cell culture management systems.

## Claims

1. A cell culture management system comprising:
a data management device;
an output data server provided in a first culture device for culturing a first kind of cells;
a customer server provided in a second culture device for culturing a second kind of cells; and
a cell management server provided in a cell management compartment for storing the second kind of cells, wherein:
the data management device is connected in bidirectional communication with each of the output data server, the customer server, and the cell management server;
the output data server comprises a transmitter for transmitting a first protocol for culturing the first kind of cells to the data management device;
the customer server comprises:
one or more transmitters for transmitting order information for obtaining the second kind of cells and location information of the second culture device to the data management device; and
a receiver for receiving a second protocol for culturing the second kind of cells from the data management device;
the data management device comprises:
a receiver for receiving the first protocol from the output data server;
a receiver for receiving the order information and the location information of the second culture device from the customer server; and
one or more memorizing units for memorizing the first protocol received from the output data server, the second protocol, and the order information and the location information of the second culture device received from the customer server; and
the cell management server comprises:
a receiver for receiving the order information and the location information of the second culture device from the data management device.

2. The cell culture management system according to Claim 1, wherein the output data server comprises a receiver for receiving a third protocol for culturing a third kind of cells from the data management device.

3. The cell culture management system according to Claim 1 or 2, wherein:
the customer server comprises a transmitter for transmitting an measured culture data obtained from a cell sensor disposed in the second culture device to the data management device; and
the data management device comprises:
a receiver for receiving the measured culture data transmitted from the customer server; and
a memorizing unit for memorizing the measured culture data received from the customer server.

4. The cell culture management system according to any one of Claims 1 to 3, wherein:
the cell management server comprises:
a memorizing unit for memorizing inventory information for cells present in the cell management compartment; and
a transmitter for transmitting the inventory information to the data management device; and
the data management device comprises:
a receiver for receiving the inventory information transmitted from the cell management server; and
a memorizing unit for memorizing the received inventory information.

5. A cell culture management system comprising:
an output data server provided in a first culture device for culturing a first kind of cells;
a customer server provided in a second culture device for culturing a second kind of cells; and
a cell management server provided in a cell management compartment where the second kind of cells are stored, wherein:
the output data server, the customer server, and the cell management server are each connected to and in bidirectional communication with a data management device;
the output data server comprises:
a transmitter for transmitting a first protocol for culturing the first kind of cells to the data management device;
the customer server comprises:
one or more transmitters for transmitting order information for obtaining the second kind of cells and location information of the second culture device to the data management device; and
a receiver for receiving a second protocol for culturing the second kind of cells from the data management device; and
the cell management server comprises:
a receiver for receiving the order information and the location information of the second culture device from the data management device.

6. The cell culture management system according to Claim 5, wherein the output data server comprises a receiver for receiving a third protocol for culturing a third kind of cells from the data management device.

7. The cell culture management system according to Claim 5 or 6, wherein:
the customer server comprises a transmitter for transmitting an measured culture data obtained from a cell sensor disposed in the second culture device to the data management device; and
the data management device comprises:
a receiver for receiving the measured culture data transmitted from the customer server; and
a memorizing unit that memorizes the measured culture data received from the customer server.

8. The cell culture management system according to any one of Claims 5 to 7, wherein:
the cell management server comprises:
a memorizing unit for memorizing inventory information for cells present in the cell management compartment; and
a transmitter for transmitting the inventory information to the data management device; and
the data management device comprises:
a receiver for receiving the inventory information transmitted from the cell management server; and
a memorizing unit for memorizing the received inventory information.
